Europäisches Patentamt

European Patent Office

Office européen des brevets

(11) Veröffentlichungsnummer: **0 139 975**
**B1**

(19)

(12) **EUROPÄISCHE PATENTSCHRIFT**

(45) Veröffentlichungstag der Patentschrift:
26.07.89

(51) Int. Cl.⁴: **A 61 L 2/04, A 61 K 35/16**

(21) Anmeldenummer: **84109915.3**

(22) Anmeldetag: **21.08.84**

(54) Verfahren zur Pasteurisierung von Humanplasma.

(30) Priorität: **26.08.83 DE 3330770**

(43) Veröffentlichungstag der Anmeldung:
**08.05.85 Patentblatt 85/19**

(45) Bekanntmachung des Hinweises auf die Patenterteilung:
**26.07.89 Patentblatt 89/30**

(84) Benannte Vertragsstaaten:
**AT BE CH DE FR GB IT LI LU NL SE**

(56) Entgegenhaltungen:
EP-A- 0 018 561
EP-A- 0 035 204
EP-A- 0 047 462
EP-A- 0 053 338
EP-A- 0 056 629
EP-A- 0 106 269
US-A- 3 686 395

(73) Patentinhaber: **BEHRINGWERKE Aktiengesellschaft,**
**Postfach 1140, D-3550 Marburg 1 (DE)**

(72) Erfinder: **Heimburger, Norbert, Prof. Dr., Sonnenhang 10,**
**D-3550 Marburg (DE)**
Erfinder: **Karges, Hermann Erich, Dr., Sonnenweg 32,**
**D-3550 Marburg (DE)**
Erfinder: **Kumpe, Gerhardt, Aspherfeld 14,**
**D-3552 Wetter (DE)**
Erfinder: **Wormsbächer, Wilfried, Blumenweg 9,**
**D-3575 Kirchhain (DE)**

(74) Vertreter: **Meyer-Dulheuer, Karl-Hermann, Dr. et al,**
**HOECHST Aktiengesellschaft Zentrale Patentabteilung**
**Postfach 80 03 20, D-6230 Frankfurt/Main 80 (DE)**

## Beschreibung

Die Erfindung betrifft ein Verfahren zur Herstellung eines pasteurisierten lipoprotein- und prothrombinfaktorenfreies Human-Plasma-Produkts sowie ein nach diesem Verfahren hergestelltes Produkt.

Frischplasma und auch fresh-frozen-plasma (FFP) wird in großen Mengen in der klinischen Routine verwendet. Hauptindikationen sind die Auffüllung des Blutvolumens zur Aufrechterhaltung des onkotischen Drucks, Blutverlust nach großen Operationen und Unfällen sowie die Behandlung der verschiedenen Formen des Schocks und die Erstversorgung von Polytraumatisierten. Für die Versorgung dieser Patienten ist nur ein Plasma indiziert, das nach Möglichkeit alle Proteine in nativer Form enthält, speziell die Enzyme und Inhibitoren des Gerinnungssystems, des Kalikrein-Kinin-Systems und der Komplement-Reaktion sowie auch die Transportproteine und Antikörper.

Ein generelles Problem bei der Anwendung von Frischplasma und auch FFP stellt das Risiko einer Hepatitis-Übertragung dar. Es ist geringer, wenn die Blutspender überwacht werden, was jedoch nur in wenigen Fällen gewährleistet werden kann.

Das Hepatitisrisiko resultiert aus zwei Ursachen: erstens sind die Testsysteme für Hepatitis B-Virus nicht genügend empfindlich, um ein Infektionsrisiko eindeutig auszuschließen; zweitens gibt es für die Nicht A/Nicht B-Hepatitis bisher noch keinen Test. Schließlich besteht auch das Risiko einer Übertragung anderer Viren wie beispielsweise Epstein-Barr und Cytomegalie, das heißt, die Anwendung einer Blutkonserve würde jeweils eine Vielzahl von relativ kostenaufwendigen Untersuchungen erfordern, abgesehen davon, daß für bestimmte Viren noch keine Methoden zur Identifizierung und Quantifizierung zur Verfügung stehen.

Dieser Stand der Entwicklung läßt erkennen, daß ein dringendes Bedürfnis für ein Verfahren besteht, das die Pasteurisierung von Plasma bei Erhaltung der Nativität der einzelnen Plasmaproteine mit ihren unterschiedlichen biochemischen Funktionen erlaubt.

Durch eine Pasteurisierung werden Viren abgetötet, wie am Beispiel von Albumin bereits gezeigt wurde. Solche Verfahren zur Pasteurisierung von Blutbestandteilen sind neben Albumin für gewisse Gerinnungsfaktoren bekannt. In der DE-A-2 916 711 ist ein Verfahren beschrieben, das erlaubt, Lösungen der Gerinnungsfaktoren II, VIII, XIII und von Antithrombin III sowie Plasminogen zu pasteurisieren, wozu Aminosäuren und Saccharide oder Zuckeralkohole zugesetzt werden. Auf diese Weise ist eine Erhitzung über 10 Stunden auf 60 °C möglich, die das Risiko einer Hepatisübertragung praktisch ausschließt.

Der Erfindung lag daher die Aufgabe zugrunde, ein Verfahren zur Pasteurisierung von Humanplasma zur Verfügung zu stellen, das auf ein natives Plasma anwendbar ist, keine großen Aktivitätsverluste der Plasmaproteine verursacht und gewährleistet, daß ein hepatissicheres Produkt resultiert.

Die Lösung der Aufgabe setzte voraus, daß man Plasma beispielsweise 10 Stunden auf 60 °C erhitzt. Nun ist jedoch bekannt, daß bereits wenige Minuten nach Erwärmen auf 60 °C das Fibrinogen und auch andere Plasmaproteine denaturieren und ausfallen. Dieses Problem konnte daher nicht in der in EP-A-18561 dargestellten Weise gelöst werden, da unter diesen Bedingungen beispielsweise Fibrinogen nicht ausreichend stabil ist.

Es war daher überraschend, daß es mit Hilfe bestimmter Zusätze von zweiwertigen Metallionen sowie Kohlenhydraten und Aminosäuren gelang, das Plasma so zu stabilisieren, daß es ohne wesentliche Protein- und Aktivitätsverluste 10 Stunden auf 60 °C erhitzt werden konnte, was in EP-A-35 204 mit einem unterschiedlichen Stabilisator nicht gezeigt wird, wenn zuvor aus tiefgefrorenem und wieder aufgetautem Human-Citratplasma die Prothrombinfaktoren (Faktor II, VII, IX und X), beispielsweise durch Adsorption an Al $(OH)_3$, $Ca_3(PO_4)_2$, wie dies aus EP-A-56 629 bekannt ist, oder DEAESephadex$^{(R)}$ sowie die labilen Proteine, besonders Lipoproteine durch Adsorption an Polyhydroxymethylen nach der EP-A-137 221 oder an ein Silicagel, beispielsweise Aerosil$^{(R)}$, oder Flotieren der Lipoproteine mit organischen Lösungsmitteln entfernt worden waren, wobei auch eine Verunreinigung durch Hepatitis B-Viren verringert wird.

Gegenstand der Erfindung ist deshalb ein Verfahren zur Gewinnung eines pasteurisierten Human-Plasma-Produkts, gekennzeichnet dadurch, daß aus Human-Citratplasma die Prothrombinfaktoren sowie Lipoproteine entfernt werden und dieses Plasmaprodukt in Anwesenheit einer Aminosäure und eines Kohlenhydrats und gegebenenfalls von Ionen eines zweiwertigen Metalls 1 Minute bis 48 Stunden auf eine Temperatur zwischen 30 °C und 100 °C erhitzt wird.

Man wird dabei aus Sicherheitsgründen möglichst ein Ausgangsmaterial einsetzen, in welchem mit einem bekannten Test, beispielsweise einem Radio Immune Assay (RIA), keine Hepatisviren nachgewiesen werden können, obwohl das beanspruchte Verfahren auch auf diesem Kriterium nicht entsprechende Ausgangsmaterialien angewendet werden kann. Andererseits garantiert ein negatives Testergebnis nicht die Virusfreiheit, wie bereits geschrieben. Zur Stabilisierung der Plasmaproteine für die Pasteurisierung werden ein Kohlenhydrat und eine Aminosäure sowie zweiwertige Metallionen, vorzugsweise Calcium- oder Magnesiumionen zugegeben und die Mischung erhitzt.

Zur Stabilisierung kommen besonders in Frage eine der Aminosäuren Glycin, α- oder β-Alanin, Hydroxyprolin, Prolin, Glutamin, α-, β- oder γ-Aminobuttersäure, vorzugsweise aber Glycin, und ein Mono- oder Oligosaccharid oder ein Zuckeralkohol, vorzugsweise Saccharose.

Die Aminosäure wird in einer Menge von 0,25 bis 3 mol/l, vorzugsweise 1 mol/l, zugegeben und es werden 35 bis 100 g, vorzugsweise 100 g Kohlenhydrat mit 100 ml der Plasmaprotein Lösung gemischt; bei der bevorzugten Arbeitsweise enthält die entstehende Lösung das Kohlenhydrat dann in einer Konzentration von 60 g/100 ml.

Zweiwertige Metallionen werden in einer Menge von 1 bis 100 mmol, vorzugsweise 15 mmol, pro Liter Lösung zugesetzt.

Es wird 1 Minute bis 48 Stunden, vorzugsweise 5–15 Stunden, auf eine Temperatur zwischen 30 °C und 100 °C, vorzugsweise 50 °C bis 70 °C, erhitzt, wobei die kürzeste Zeit der höchsten Temperatur zuzuordnen ist und umgekehrt.

Danach können die Stabilisatoren abgetrennt werden. Es können dann eine Gleichgewichtsdialyse, eine Konzentrierung auf einem Ultrafilter, eine Sterilfiltration und/oder eine Abfüllung folgen.

Wie an den folgenden Beispielen gezeigt wird, gelang es durch das beschriebene Verfahren, Plasma unter Erhaltung der biologischen Aktivität der wichtigsten Proteine einschließlich der Gerinnungsfaktoren und ihrer Inhibitoren zu pasteurisieren. Darüber hinaus wurde gefunden, daß auch die Antikörperwirkung der Immunglobuline nach der Pasteurisierung erhalten ist. Mit den in der Proteinchemie üblichen Analysenmethoden wurden keine Hinweise dafür erhalten, daß es während der Erhitzung zur Fragmentierung von Proteinen kommt.

Ein besonders geeignetes Verfahren zur Herstellung eines hepatissicheren, nativen Humanplasma-Präparates ist das folgende: Adsorption der Prothrombinfaktoren an Al(OH)$_3$, Filtration des adsorbierten Plasmas über eine Säule aus Polyhydroxymethylen (PHM) und Stabilisierung des Säuleneluats durch Zugabe von 100 g Saccharose auf 100 ml Eluat, 1 mol Glycin und 15 mmol Calciumionen pro Liter Lösung. Die vor der Erhitzung entfernten Prothrombinfaktoren können vom Al(OH)$_3$ eluiert und, getrennt pasteurisiert, dem Plasma anschließend wieder zugesetzt werden.

Beispiel 1

Ausgangsmaterial: 500 ml tiefgefrorenes Human-Citratplasma. Das Plasma wurde bei 20 °C aufgetaut und zur Entfernung der Prothrombinfaktoren 2 × mit 25 ml einer 1%igen Al(OH)$_3$-Suspension 15 Minuten gerührt, anschließend zentrifugiert. Der Al(OH)$_3$-Rückstand wurde verworfen.

Stabilisierung und Pasteurisierung: Zu 500 ml des adsorbierten Plasmas wurden 7,5 ml einer CaCl$_2$-Lösung mit 1 mol/l pipettiert, so daß die Ca$^{2+}$-Endkonzentration 15 mmol/l betrug; anschließend wurden unter weiterem Rühren und Erwärmen 500 g Saccharose zugesetzt und, nachdem sich diese vollständig gelöst hatte, 37,5 g Glycin (1 mol/l). Der pH-Wert wurde dann mit 2 N NaOH auf 7,3 eingestellt und bis zur Konstanz nachgestellt.

Durch die Zusätze vergrößerte sich das Volumen von 500 ml auf 850 ml einer klaren viskosen Lösung, die im Wasserbad 10 Stunden bei 60 °C erhitzt wurde. Die Lösung war auch nach der Erhitzung klar.

Entfernung der Stabilisatoren: Nach Erkalten der pasteurisierten Lösung wurde diese mit einem Citrat-NaCl-Puffer (0,01 mol/l Tri-Na-Citrat, pH 7,5; 0,06 mol/l NaCl) auf 2500 ml verdünnt, auf einem Ultrafilter gegen 5 l eines Puffers derselben Zusammensetzung dialysiert und auf 500 ml konzentriert. Nach Erreichen des Plasma-Ausgangsvolumens (500 ml) wurde erneut auf 2500 ml verdünnt und wiederum gegen 5 l frischen Puffers dialysiert. Nach Erreichen des Dialyse-Gleichgewichtes und Konzentrieren auf 500 ml wurde die Filtration abgebrochen. Als Endprodukt wurde eine durch Lipoproteine leicht getrübte Lösung erhalten.

Das pasteurisierte Plasma wurde 1 Stunde bei 20 °C und 30.000 × g ultrazentrifugiert, klär- und anschließend über SM-Filter sterilfiltriert, zu 50 ml in 100 ml Infusionsflaschen abgefüllt und lyophilisiert. Die Tabelle 1 enthält Eiweiß- und Funktionsbestimmungen der biologisch wichtigsten Proteine vor und nach Pasteurisierung, Lyophilisation und Rekonstitution in aqua injectabilia.

Beispiel 2

Ausgangsmaterial: 500 ml gepooltes frisches Human-Citratplasma wurde zur Entfernung der Prothrombinfaktoren bei 20 °C mit 6 g nutschenfeuchtem, in Citrat-NaCl-Puffer (0,02 mol/l Tri-Na-Citrat-Lösung, pH 7,5; 0,06 mol/l NaCl) äquilibrierten DEAE-Sephadex A-50 versetzt, 30 min lang gerührt und das DEAE-Sephadex durch Zentrifugation abgetrennt. Der Überstand wurde zur Adsorption der Lipoproteine und Hepatitis B-Viren über eine Säule mit 500 ml Polyhydroxymethylen geleitet, das mit einer Lösung enthaltend 0,01 mol/l Tri-Na-Citrat, pH 7,5 und 0,06 mol/l NaCl äquilibriert worden war. Nach Säulenpassage fielen 550 ml klares, lipoproteinfreies Plasma an.

Stabilisierung und Pasteurisierung: Zu 550 ml delipidisiertem Humanplasma wurden 8,25 ml einer CaCl$_2$-Lösung mit 1 mol/l zugesetzt und anschließend unter Rühren und Erwärmen auf 30 °C 550 g Saccharose. Nachdem sich die Saccharose aufgelöst hatte, wurden 41,5 g (1 mol/l) Glycin in die Lösung eingerührt. Der pH-Wert wurde mit 2 N NaOH auf pH 7,3 eingestellt und bis zur Konstanz reguliert.

Die so stabilisierte, klare, viskose Lösung (930 ml) wurde in einem Wasserbad 10 Stunden bei 60 °C gehalten. Die Lösung war auch nach der Erhitzung klar.

Entfernung der Stabilisatoren: Die erkaltete Lösung wurde mit einem Citrat-NaCl-Puffer (0,01 mol/l Tri-Na-Citrat, pH 7,5; 0,06 mol/l NaCl) auf 2750 ml verdünnt und auf einem Ultrafilter gegen 5 l eines Puffers derselben Zusammensetzung dialysiert und auf 500 ml konzentriert. Es wurde erneut verdünnt und gegen frischen Puffer dialysiert. Nach Erreichen des Dialysegleichgewichts und des Endvolumens von 500 ml resultierte eine klare Lösung, die über SM-Filter klär- und sterilfiltriert, zu 50 ml in 100 ml Infusionsflaschen abgefüllt und lyophilisiert wurde.

Die Konzentration und Aktivität ausgewählter Plasmaproteine vor und nach Pasteurisierung enthält Tabelle 1.

## Tabelle 1:

Gesamteiweiß-, Fibrinogen- und Fibronectin-Gehalte sowie Aktivitäten von ausgewählten Gerinnungsfaktoren und Inhibitoren vor und nach Pasteurisierung von Humanplasma

AT III = Antithrombin III / AP = $\alpha_2$-Antiplasmin / HMW = high molecular weight kininogen / Apo = Apolipoprotein A bzw. B

| Behandlung des Plasmas | Protein mg/ml | Fibri-nogen mg/dl | Fibro-nectin mg/dl | F V % der Norm* | F VIII % der Norm | F XI % der Norm | F XII % der Norm | HMW-Ki-ninogen % der Norm | AP % der Norm | AT III % der Norm | Apo A mg/dl | Apo B mg/dl |
|---|---|---|---|---|---|---|---|---|---|---|---|---|
| unbehandelt (vor Pasteurisierung) | 44 | 212 | 25 | 60 | 70 | 100 | 100 | 90 | 93 | 92 | 134 | 79 |
| nach Pasteurisierung gem. Beispiel 1 | 39 | 169 | 19,3 | 13 | 34 | 21 | 60 | 55 | 82 | 77 | 98 | 81 |
| nach Pasteurisierung gem. Bespiel 2 | 35 | 224 | 19,3 | 5 | 27 | 36 | 50 | 36 | 67 | 83 | 4 | 10 |

*«Norm» = Gehalt eines Plasmapools von mindestens 40 gesunden männlichen Spendern (= 100%)

In Tabelle 2 sind die Antikörper-Aktivitäten vor und nach Erhitzen zusammengestellt.

## Tabelle 2:

Antikörper-Titer im Plasma vor und nach Pasteurisierung gemäß Beispiel 2

| Plasma | Röteln | Masern | Tetanus (IE/ml) |
|---|---|---|---|
| vor Pasteurisierung | 1:150 | 1:64 | 1 |
| nach Pasteurisierung | 1:120 | 1:64 | 1 |

## Patentansprüche

1. Verfahren zur Gewinnung eines pasteurisierten Human-Plasma-Produkts, gekennzeichnet dadurch, daß aus Human-Citratplasma die Prothrombinfaktoren sowie Lipoproteine entfernt werden und dieses Plasmaprodukt in Anwesenheit einer Aminosäure und eines Kohlenhydrats und gegebenenfalls von Ionen eines zweiwertigen Metalls 1 Minute bis 48 Stunden auf eine Temperatur zwischen 30°C und 100°C erhitzt wird.

2. Verfahren nach Anspruch 1, dadurch gekennzeichnet, daß die Prothrombinfaktoren sowie labile Proteine mittels eines Polyhydroxymethylens entfernt werden, das einen aufgepfropften oxethylierten Alkohol oder eine aufgepfropfte oxethylierte Carbonsäure enthält.

3. Verfahren nach Anspruch 1, dadurch gekennzeichnet, daß die Aminosäure Glycin, alpha- oder beta-Alanin, Hydroxyprolin, Prolin, Glutamin, alpha-, beta- oder gamma-Aminobuttersäure in einer Konzentration von 0,25–3 mol/l ist.

4. Verfahren nach Anspruch 1, dadurch gekennzeichnet, daß das Kohlenhydrat Saccharose in einer Konzentration von 35–60 g/100 ml Lösung ist.

5. Verfahren nach Anspruch 1, dadurch gekennzeichnet, daß die zweiwertigen Metallionen Ca-Ionen in einer Konzentration von 1–100 mmol/l sind.

6. Verfahren nach einem der Ansprüche 1 bis 5, dadurch gekennzeichnet, daß 5–15 Stunden auf eine Temperatur von 50–70 °C erhitzt wird.

7. Pasteurisiertes Human-Plasma-Produkt erhältlich nach dem Verfahren gemäß Anspruch 1.

## Claims

1. A process for obtaining a pasteurized human plasma product, which comprises removing the prothrombin factors and lipoproteins from human citrated plasma, and heating this plasma product in the presence of an amino acid and a carbohydrate and, if appropriate, ions of a divalent metal for 1 minute to 48 hours at a temperature between 30 °C and 100 °C.

2. A process as claimed in claim 1, wherein the prothrombin factors and the labile proteins are removed by means of a polyhydroxymethylene which contains a grafted-on oxethylated alcohol or a grafted-on oxethylated carboxylic acid.

3. The process as claimed in claim 1, wherein the amino acid glycine, alpha- or beta-alanine, hydroxyproline, proline, glutamine or alpha-, beta- or gamma-amino butyric acid is in a concentration of 0.25–3 moles/liter.

4. The process as claimed in claim 1, wherein the carbohydrate sucrose is in a concentration of 35–60 g/100 ml of solution.

5. The process as claimed in claim 1, wherein the divalent metal ions are Ca ions in a concentration of 1–100 mmoles/liter.

6. The process as claimed in any one of claims 1 to 5, wherein heating is carried out at a temperature of 50–70 °C for 5–15 hours.

7. A pasteurized human plasma product obtainable by the process as claimed in claim 1.

## Revendications

1. Procédé d'obtention d'un produit plasmatique humain pasteurisé, caractérisé en ce que l'on élimine du plasma humain citraté les facteurs de prothrombine ainsi que les lipoprotéines et en ce que l'on chauffe ce produit plasmatique pendant 1 minute à 48 heures à une température comprise entre 30 °C et 100 °C en présence d'un aminoacide et d'un hydrate de carbone et éventuellement d'ions d'un métal divalent.

2. Procédé selon la revendication 1, caractérisé en ce que l'on élimine les facteurs de prothrombine ainsi que les protéines labiles au moyen d'un polyhydroxyméthylène qui comporte un alcool oxéthylé greffé ou un acide carboxylique oxéthylé greffé.

3. Procédé selon la revendication 1, caractérisé en ce que l'aminoacide est la glycine, l'alpha ou béta-alanine, l'hydroxyproline, la proline, la glutamine, l'acide alpha-, béta- ou gamma-aminobutyrique à une concentration de 0,25 à 3 moles/l.

4. Procédé selon la revendication 1, caractérisé en ce que l'hydrate de carbone est le saccharose à une concentration de 35 à 60 g/100 ml de solution.

5. Procédé selon la revendication 1, caractérisé en ce que les ions de métal divalent sont des ions Ca à une concentration de 1 à 100 mmoles/l.

6. Procédé selon l'une des revendications 1 à 5, caractérisé en ce que l'on chauffe pendant 5 à 15 heures à une température de 50 à 70 °C.

7. Produit plasmatique humain pasteurisé pouvant être obtenu par le procédé selon la revendication 1.